# FASCICULE DE BREVET EUROPEEN

(11) **EP 0 864 304 B1**
(45) Date de publication et mention de la délivrance du brevet: **31.07.2002**
(21) Numéro de dépôt: 98420040.2
(22) Date de dépôt: 05.03.1998
(51) Int. Cl.: A61F 2/42

(54) **Prothèse de cheville**
Knöchelprothese
Ankle prosthesis

(30) Priorité: 10.03.1997 FR 9703041
(43) Date de publication de la demande: 16.09.1998
(73) Titulaire: TORNIER SA, 38330 Saint-Ismier (FR)
(72) Inventeur: Bonnin, Michel, 69340 Francheville (FR); Colombier, Jean-Alain, 31130 Balma (FR); Judet, Thierry, 92410 Ville D'Avray (FR); Tornier, Alain, 38330 Saint Ismier (FR)
(74) Mandataire: Myon, Gérard Jean-Pierre

(56) Documents cités:
- EP-A- 0 201 651
- DE-A- 2 830 354
- FR-A- 2 692 776
- FR-A- 2 697 155
- FR-A- 2 728 783
- FR-A- 2 730 157
- US-A- 3 987 500
- US-A- 5 326 365

## Description

La présente invention concerne une prothèse partielle et une prothèse totale de cheville.

On sait que le tibia et le péroné, réunis par deux puissants ligaments, présentent ensemble trois surface articulaires, l'une médiane, coopérant avec la poulie astragalienne et deux autres, latérales, disposées en regard des joues astragaliennes. De nombreuses propositions ont déjà été faites en ce qui concerne la forme des surfaces articulaires principales entre le composant tibial et le composant astragalien d'une prothèse tibio-astragalienne de cheville.

Par contre, une attention insuffisante est souvent portée à la réalisation des surfaces articulaires latérales et en particulier de celles mettant en jeu le péroné et l'astragale ou talus.

Le but de l'invention est précisément de s'attacher à ce problème et de proposer une prothèse permettant la reconstruction du versant articulaire de la malléole externe de la cheville.

Dans le cas de la pose d'une prothèse de cheville, deux hypothèses se présentent :
- soit la cheville n'est que partielle endommagée, auquel cas il est suffisant de poser une prothèse partielle de cheville ;
- soit la cheville est très endommagée, auquel cas il est nécessaire de procéder à la pose d'une prothèse complète de cheville.

Dans le premier cas, c'est-à-dire celui d'une prothèse partielle, les éléments prothétiques doivent être compatibles avec les surfaces d'articulation osseuses qui sont conservées. Dans le second cas, c'est-à-dire celui d'une prothèse totale, une reconstruction totale de ces surfaces d'articulation est envisageable.

Par le document US-A-3,987,500 on connaît une prothèse totale de cheville dans laquelle un implant malléolaire comprenant une surface d'articulation concave est destiné à coopérer avec une surface convexe d'une partie astragalienne de la prothèse. La géométrie de la surface de l'implant malléolaire est telle qu'elle ne pourrait pas coopérer avec une astragale non équipée d'un élément prothétique. En d'autres termes la structure connue n'est pas transposable à une prothèse partielle de cheville, sauf à intervenir sur l'astragale ce qui n'est pas admissible pour le patient. En outre, la géométrie des surfaces d'articulation de l'élément astragalien et de l'implant malléolaire ne permet pas d'obtenir un guidage efficace de l'articulation de ces deux pièces.

L'invention résoud ces problèmes, tant en ce qui concerne une prothèse partielle qu'en ce qui concerne une prothèse totale de cheville.

Dans cet esprit, l'invention concerne une prothèse partielle de cheville caractérisée en ce qu'elle comprend uniquement un implant malléolaire apte à être installé à la partie inférieure du péroné de façon à constituer une surface d'articulation du péroné sur une partie externe de l'astragale, cet implant comportant, d'une part, des moyens de fixation dans le péroné et, d'autre part, une surface bombée apte à coopérer avec la surface articulaire externe de l'astragale.

Grâce à l'invention, le resurfaçage latéral partiel de l'articulation de la cheville est rendu possible en n'intervenant que sur le péroné. Ainsi, les mouvements relatifs obtenus entre le tibia, l'astragale et le péroné sont compatibles avec les mouvements naturels d'une cheville ne comportant pas de prothèse. Il n'en résulte donc pas de gêne particulière pour le patient.

'Dans le cas d'une prothèse totale de cheville, l'invention concerne une prothèse comprenant, une pièce astragalienne comportant des moyens de fixation sur l'astragale, une surface articulaire supérieure et au moins une surface articulaire latérale dirigée vers le péroné, cette prothèse comprenant également un implant malléolaire, caractérisée en ce que cette surface articulaire latérale présente une double courbure, convexe suivant une direction sensiblement verticale, cet implant comportant, d'une part, des moyens de fixation dans le péroné et, d'autre part, une surface bombée apté à coopérer avec la surface articulaire de la pièce astragalienne.

La prothèse totale de l'invention permet un guidage efficace de l'articulation du péroné par rapport à l'astragale grâce à la forme particulière de la surface articulaire latérale de la pièce astragalienne et de la surface bombée de l'implant malléolaire, cette articulation étant compatible avec les mouvements naturels de la cheville.

Selon un premier mode de réalisation de l'invention, les moyens de fixation comprennent une queue apte à pénétrer dans un perçage du péroné, cette queue étant pourvue d'une multiplicité de collerettes de coincement à l'intérieur du perçage.

Selon un second mode de réalisation avantageux de l'invention, les moyens de fixation comprennent une multiplicité de dents prévues à l'arrière de la surface bombée, ces dents étant destinées à être impactées dans le péroné.

Selon un troisième mode de réalisation avantageux de l'invention, les moyens de fixation consistent en un filetage externe formé sur une partie arrière de l'implant qui est en forme de plot ou de bouton. Ce filetage externe permet un vissage de l'implant dans ou à travers le péroné.

Ces différentes variantes de moyens de fixation, utilisables avec la prothèse partielle et la prothèse totale de . l'invention, permettent toutes une fixation efficace dé l'implant par rapport au péroné alors que la seule surface accessible depuis l'extérieur de l'os, une fois l'implant monté, est la surface bombée destinée à constituer une surface articulaire.

Dans le cas de la prothèse totale et selon une première variante avantageuse de l'invention, alors que la surface articulaire supérieure présente, vue en plan, une courbure dont la concavité est dirigée vers la malléole interne, les centres de courbure des surfaces articulaires supérieure et latérale dans une telle vue en plan, sont sensiblement situés sur le même rayon moyen ou médian.

De même toujours dans le cas de la prothèse totale et selon une autre variante avantageuse de réalisation de l'invention et alors que la surface articulaire supérieure présente, en vue en plan, une courbure dont la concavité est dirigée vers la malléole interne, le centre de courbure de la surface articulaire latérale est décalé vers l'avant par rapport au centre de courbure de la surface articulaire supérieure.

L'invention sera mieux comprise et d'autres avantages de celle-ci apparaîtront plus clairement à la lumière de la description qui va suivre de quatre modes de réalisation d'une prothèse de cheville conforme à son principe, donnée uniquement à titre d'exemple et faite en référence aux dessins annexés dans lesquels :
- la figure 1 est une vue de face d'une prothèse partielle de cheville conforme à un premier mode de réalisation de l'invention ;
- la figure 2 est une vue de face d'une prothèse partielle de cheville conforme à un second mode de réalisation de l'invention ;
- la figure 3 est une vue en perspective d'un implant malléolaire utilisé dans le mode de réalisation de la figure 2 ;
- la figure 4 est une vue de face d'une prothèse totale de cheville conforme à un troisième mode de réalisation de l'invention ;
- la figure 5 est une vue de dessus des éléments prothétiques de la prothèse de la figure 4 et
- la figure 6 est une vue analogue à la figure 5 pour une prothèse conforme à un quatrième mode de réalisation de l'invention.

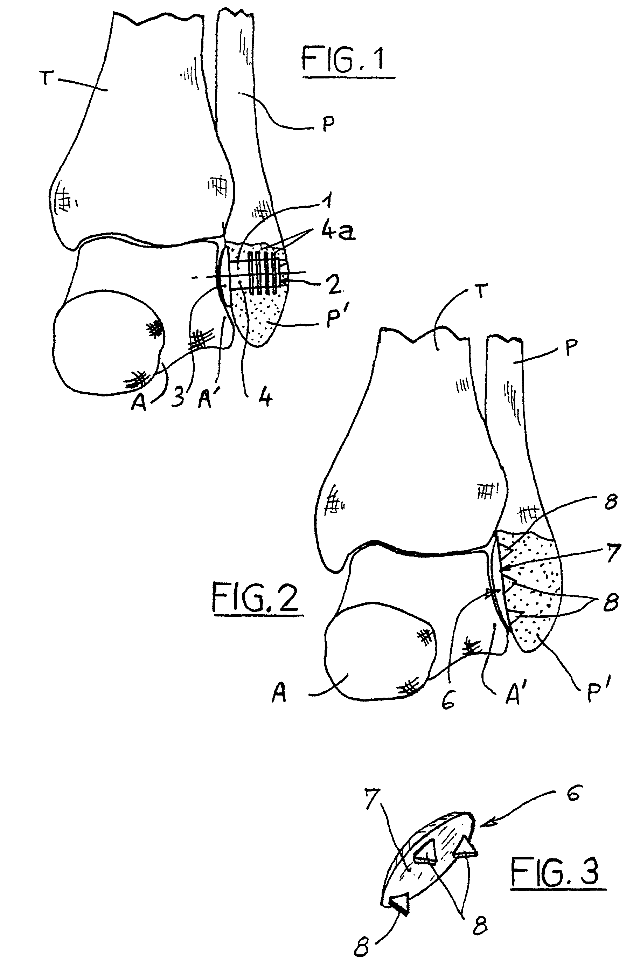

A la figure 1, un tibia T et un péroné P sont articulés par rapport à l'astragale A d'une cheville. Conformément à l'invention, un implant 1 est inséré dans un perçage 2 de la partie inférieure P' du péroné P. Cet implant 1 comprend une tête bombée 3, en forme de calotte sphérique, dont le rayon de courbure est sensiblement égal à celui de la partie malléolaire externe ou joue A' de l'astragale A. La tête 3 pourrait également avoir toute autre forme bombée proche de la forme anatomique de la malléole.

L'implant 1 comprend aussi une queue 4 destinée à pénétrer à l'intérieur du perçage 2. La queue 4 est munie de plusieurs collerettes externes 4a qui forment un moyen de retenue ou de coincement de la queue 4 à l'intérieur de l'orifice 2.

Dans le second mode de réalisation de l'invention représenté à la figure 2, un implant 6, plus particulièrement visible à la figure 3, comprend une partie bombée 7 sur la face arrière de laquelle sont prévues trois dents 8 destinées à être impactées dans la partie inférieure P' du péroné P de la cheville à équiper. Comme précédemment, l'implant 6 constitue une surface d'articulation du péroné P sur la partie externe A' de l'astragale A.

Ce mode de réalisation présente, par rapport à celui de la figure 1, l'avantage qu'il n'est pas nécessaire de réaliser dans la partie inférieure P' du péroné P un perçage ou avant-trou préalablement à la mise en place de l'implant 6.

Dans les deux modes de réalisation de l'invention précédemment décrits, la prothèse comprend uniquement l'implant malléolaire 1 ou 6, de sorte que sa mise en place nécessite une intervention minimale sur la cheville. La forme bombée de l'implant 1 ou 6 lui permet d'être compatible avec la joue naturelle A' de l'astragale.

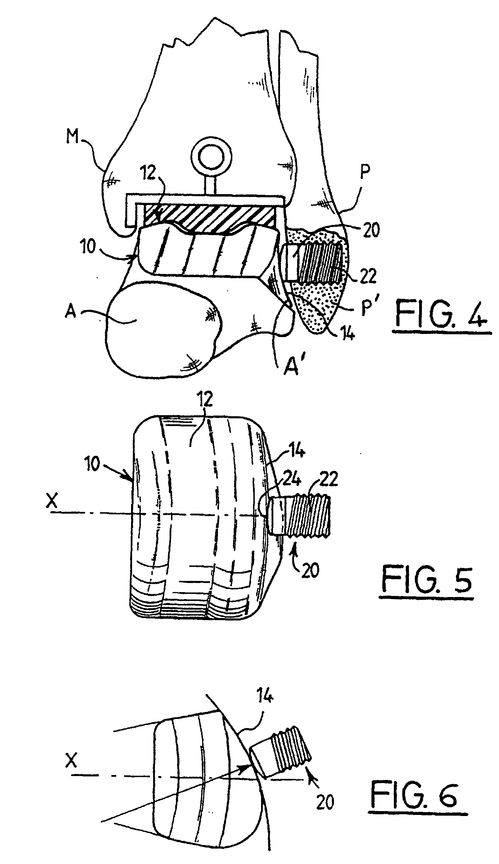

Le troisième mode de réalisation de l'invention représenté aux figures 4 et 5 montre un implant 20, en forme de plot ou de bouton, qui est fixé dans la partie inférieure du péroné et qui comporte une partie arrière munie d'un filetage externe 22 et une surface frontale bombée 24 assurant le contact avec la surface articulaire astragalienne A' en regard. Cet élément peut être fixé dans l'extrémité inférieure du péroné par vissage à partir de la face interne de l'os, ou bien par vissage à partir de la face externe de cet os, ce qui peut en faciliter la mise en place. Le vissage permet également de régler la position de la surface bombée 24 par rapport à l'os.

Les figures 4 et 5 représentent l'utilisation d'une prothèse totale de cheville conforme à l'invention. On voit sur le dessin une partie d'une prothèse de cheville, à savoir la partie astragalienne 10 de cette prothèse, munie de moyens connus et non représentés permettant de la fixer de toute façon convenable sur l'astragale A.

Ce composant astragalien délimite une surface articulaire supérieure 12 présentant, vue en plan, une courbure dont la concavité est dirigée vers la malléole interne M, ou vers l'intérieur du pied du patient équipé d'une telle prothèse. La forme de cette surface articulaire supérieure ne sera pas décrite plus en détails.

La pièce 10 comporte de plus une surface articulaire latérale 14 disposée en regard de la partie inférieure du péroné P et constituant la partie ou joue externe A' de l'astragale A. Cette joue ou partie externe A' est destinée à coopérer avec la surface bombée 24 de l'implant 20.

La surface articulaire latérale présente une double courbure : une courbure convexe suivant une direction avant-arrière et une courbure concave suivant une direction sensiblement verticale. Cette double courbure de la surface articulaire 14 lui permet d'être en contact glissant avec la surface bombée 24 de l'implant 20 dans toutes les positions de la cheville et d'assurer ainsi un guidage efficace du péroné par rapport à l'astragale.

Dans le mode de réalisation représenté aux figures 4 et 5, la courbure de la surface 14 en projection sur le plan de la figure est sensiblement la même que celle de la surface articulaire supérieure 12. A tout le moins, les centres de courbure de ces deux surfaces sont sensiblement placés sur le même axe X correspondant à un rayon moyen ou médian de ces surfaces.

Dans la variante de la figure 6, par contre, le centre de courbure de la surface latérale 14 est décalé vers l'avant, procurant ainsi une surface articulaire latérale excentrée par rapport à la surface articulaire supérieure.

Une telle forme est de nature à assurer un meilleur guidage latéral lorsque l'articulation entre les surfaces articulaire tibiale et astragalienne autorise un certain débattement latéral.

Les implants des différents modes de réalisation décrits peuvent être monobloc, en matériau plastique, en céramique ou en métal, ou être réalisés en deux parties, une partie métallique d'ancrage dans l'os et un revêtement ou insert, par exemple en polyéthylène haute densité, assurant une fonction de friction avec des forces de frottement faibles lorsqu'on l'associe à une prothèse de cheville munie d'une joue astragalienne externe métallique.

Des moyens de fixation des implants 1, 6 ou 20 autres que ceux représentés peuvent également être utilisés sans sortir du cadre de l'invention.

Dans les quatre variantes envisagées, l'utilisation d'un implant bombé, rapporté sur le péroné et correctement positionné, permet d'atteindre l'objectif recherché, à savoir un guidage latéral amélioré de l'articulation. Ce résultat est de plus atteint par des moyens simples et de mise en place relativement aisée.

Bien entendu, les prothèses des modes de réalisation des figures 1 à 3 pourraient également être utilisées avec des prothèses de cheville tibio-astragaliennes, c'est-à-dire totales.

## Revendications

1. Prothèse partielle de cheville **caractérisée en ce qu'**elle comprend uniquement un implant malléolaire (1, 6, 20) apte à être installé à la partie inférieure (P') du péroné (P) de façon à constituer une surface d'articulation du péroné sur une partie externe (A') de l'astragale (A), ledit implant comportant, d'une part, des moyens (4, 4a, 8, 22) de fixation dans le péroné (P) et, d'autre part, une surface bombée (3, 7, 24) apte à coopérer avec la surface articulaire externe (A') de l'astragale.

2. Prothèse totale de cheville comprenant une pièce astragalienne (10) comportant des moyens de fixation sur l'astragale, une surface articulaire supérieure (12) et au moins une surface articulaire latérale (14) dirigée vers le péroné, ladite prothèse comprenant également un implant malléolaire (1, 6, 20), **caractérisée en ce que** ladite surface articulaire latérale présente une double courbure, convexe suivant une direction avant-arrière et concave suivant une direction sensiblement verticale, ledit implant comportant, d'une part, des moyens (4, 4a, 8, 22) de fixation dans le péroné (P) et, d'autre part, une surface bombée (3, 7, 24) apte à coopérer avec ladite surface articulaire latérale (14, A') de ladite pièce astragalienne (10).

3. Prothèse de cheville selon l'une des revendications 1 ou 2, **caractérisée en ce que** lesdits moyens de fixation dudit implant (1) comprennent une queue (4) apte à pénétrer dans un perçage (2) du péroné (P), ladite queue étant pourvue d'une multiplicité de collerettes (4a) de coincement à l'intérieur dudit perçage.

4. Prothèse de cheville selon l'une des revendications 1 ou 2, **caractérisée en ce que** lesdits moyens de fixation dudit implant (6) comprennent une multiplicité de dents (8) prévues à l'arrière de ladite surface bombée (7), lesdites dents étant destinées à être impactées dans le péroné (P).

5. Prothèse de cheville selon l'une des revendications 1 ou 2, **caractérisée en ce que** lesdits moyens de fixation consistent en un filetage externe (22) formé sur une partie arrière dudit implant (20) qui est en forme de plot ou de bouton, ledit filetage permettant le réglage de la position de ladite surface bombée par rapport à l'os.

6. Prothèse totale de cheville selon la revendication 2, **caractérisée en ce que** la surface articulaire supérieure (12) présente, vue en plan, une courbure dont la concavité est dirigée vers la malléole interne, et **en ce que** les centres de courbure des surfaces articulaires supérieure (12) et latérale (14), dans une telle vue en plan, sont sensiblement situés sur le même rayon moyen ou médian (X).

7. Prothèse totale de cheville selon la revendication 2, **caractérisée en ce que** la surface articulaire supérieure (12) présente, vue en plan, une courbure dont la concavité est dirigée vers la malléole interne, et **en ce que** le centre de courbure de la surface articulaire latérale (14) est décalé vers l'avant par rapport au centre de courbure de la surface articulaire supérieure (12).

## Patentansprüche

1. Fußgelenksteilprothese, **dadurch gekennzeichnet, dass** sie nur ein Knöchelimplantat (1, 6, 20) umfasst, das am unteren Teil (P') des Wadenbeins (P) angebracht wird, so dass es eine Gelenkfläche des Wadenbeins auf einem äußeren Teil (A') des Sprungbeins (A) bildet, wobei das Implantat einerseits Mittel (4, 4a, 8, 22) zur Befestigung im Wadenbein (P) und andererseits eine gewölbte Oberfläche (3, 7, 24) aufweist, die in der Lage ist, mit der äußeren Gelenkoberfläche (A') des Sprungbeins zusammenzuwirken.

2. Fußgelenksvollprothese mit einem sprungbeinseitigen Teil (10) mit einem Mittel zur Befestigung am Sprungbein, einer oberen Gelenkfläche (12) und zumindest einer seitlichen Gelenkfläche (14), die zum Wadenbein gerichtet ist, wobei die Prothese ebenso ein Knöchelimplantat (1, 6, 20) umfasst, **dadurch gekennzeichnet, dass** die seitliche Gelenkfläche eine doppelte Krümmung aufweist, konvex längs einer Richtung von vorn nach hinten und konkav längs einer im wesentlichen vertikalen Richtung, wobei das Implantat einerseits Mittel (4, 4a, 8, 22) zur Befestigung im Wadenbein (P) und andererseits eine gewölbte Fläche (3, 7, 24) aufweist, die in der Lage ist, mit der seitlichen Gelenkoberfläche (14, A') des Sprungbeinteils (10) zusammenzuwirken.

3. Knöchelprothese nach einem der Ansprüche 1 oder 2, **dadurch gekennzeichnet, dass** die Mittel zur Befestigung des Implantats (1) einen Schaft (4) umfassen, der daran ausgebildet ist, in eine Bohrung (2) des Wadenbeins (P) einzudringen, wobei der Schaft mit einer Mehrzahl von Bünden (4a) zur Verklemmung im Inneren der Bohrung versehen ist.

4. Knöchelprothese nach einem der Ansprüche 1 oder 2, **dadurch gekennzeichnet, dass** die Befestigungsmittel des Implantats (6) eine Vielzahl von Zähnen (8) umfassen, die auf der Rückseite der gewölbten Fläche (7) vorgesehen sind, wobei die Zähne dafür bestimmt sind, in das Wadenbein (P) eingeschlagen zu werden.

5. Knöchelprothese nach einem der Ansprüche 1 oder 2, **dadurch gekennzeichnet, dass** die Befestigungsmittel aus einem Außengewinde (22) bestehen, das auf einem rückwärtigen Teil des Implantats (20) ausgebildet ist, das Stiftform oder Knopfform hat, wobei das Gewinde die Einstellung der Position der gewölbten Oberfläche bezüglich des Knochens gestattet.

6. Knöchelvollprothese nach Anspruch 2, **dadurch gekennzeichnet, dass** die obere Gelenkfläche (12) in Aufsicht eine Krümmung aufweist, deren Konkavität zum inneren Knöchel gerichtet ist, und dass die Krümmungsmittelpunkte der oberen (12) und seitlichen (14) Gelenkfläche in einer derartigen Aufsicht im wesentlichen auf dem gleichen Mittel- oder Medianstrahl (X) liegen.

7. Knöchelvollprothese nach Anspruch 2, **dadurch gekennzeichnet, dass** die obere Gelenkfläche (12) in Aufsicht eine Krümmung aufweist, deren Konkavität zum inneren Knöchel gerichtet ist, und dass der Krümmungsmittelpunkt der seitlichen Gelenkfläche (14) in Bezug zum Krümmungsmittelpunkt der oberen Gelenkfläche (12) nach vorne verschoben ist.

## Claims

1. Partial ankle prosthesis **characterised in that** it comprises solely a malleolar implant (1, 6, 20) which can be installed on the lower part (P') of the fibula (P) such as to constitute a surface of articulation of the fibular on an outer part (A') of the astragalus (A), the said implant comprising firstly means (4, 4a, 8, 22) for securing in the fibula (P) and secondly a curved surface (3, 7, 24) which can co-operate with the outer articular surface (A') of the astragalus.

2. Full ankle prosthesis comprising an astragalar part (10) comprising means for securing on the astragalus, an upper articular surface (12) and at least one lateral articular surface (14) which faces the fibula, the said prosthesis also comprising a malleolar implant (1, 6, 20), **characterised in that** the said lateral articular surface has a double curvature, which is convex in a front-rear direction and is concave in a substantially vertical direction, the said implant comprising firstly means (4, 4a, 8, 22) for securing in the fibula (P) and secondly a curved surface (3, 7, 24) which can co-operate with the said lateral articular surface (14, A') of the said astragalar part (10).

3. Ankle prosthesis according to claim 1 or claim 2, **characterised in that** the said means for securing the said implant (1) comprise a tail (4) which can penetrate in a bore (2) in the fibula (P), the said tail being provided with a plurality of collars (4a) for wedging inside the said bore.

4. Ankle prosthesis according to claim 1 or claim 2, **characterised in that** the said means for securing the said implant (6) comprise a multiplicity of teeth (8) which are provided to the rear of the said curved surface (7), the said teeth being designed to be impacted in the fibula (P).

5. Ankle prosthesis according to claim 1 or claim 2, **characterised in that** the said means for securing consist of an outer thread (22) formed on a rear part of the said implant (20) which is in the form of a stud or peg, the said thread permitting adjustment of the position of the said curved surface relative to the bone.

6. Full ankle prosthesis according to claim 2, **characterised in that** in plan view, the upper articular surface (12) has a curvature, the concavity of which faces the inner malleolus, and **in that** in a plan view of this type, the centres of curvature of the upper (12) and lateral (14) articular surfaces are substantially situated on the same median or mean radius (X).

7. Full ankle prosthesis according to claim 2, **characterised in that** in plan view, the upper articular surface (12) has a curvature, the concavity of which faces the inner malleolus, and **in that** the centre of curvature of the lateral articular surface (14) is offset forwards relative to the centre of curvature of the upper articular surface (12).
